# FASCICULE DE BREVET EUROPEEN

(11) **EP 1 071 410 B1**
(45) Date de publication et mention de la délivrance du brevet: **10.07.2002**
(21) Numéro de dépôt: 99915799.3
(22) Date de dépôt: 13.04.1999
(51) Int. Cl.: A61K 31/045, A61K 9/08

(54) **COMPOSITION AQUEUSE IONIQUE CONTENANT DU LEVOMENTHOL**
IONISCHE WÄSSERIGE ZUSAMMENSETZUNG ENTHALTEND LEVOMENTHOL
IONIC AQUEOUS COMPOSITION CONTAINING LEVOMENTHOL

(30) Priorité: 14.04.1998 FR 9804809
(43) Date de publication de la demande: 31.01.2001
(73) Titulaire: P.N. Gerolymatos S.A., 14568 Kryoneri Athènes (GR)
(72) Inventeur: P.N. Gerolymatos S.A., 14568 Kryoneri Athènes (GR)
(74) Mandataire: Giraud, Françoise
(86) Numéro de dépôt international: FR9900860
(87) Numéro de publication internationale: WO9952520

(56) Documents cités:
- WO-A-95/13810
- FR-A- 2 741 535
- SEKIZAWA S. ET AL: "Nasal receptors responding to cold and L-menthol airflow in the guinea pig" RESPIR. PHYSIOL., vol. 103, no. 3, 1996, pages 211-219, XP002089539
- SU, XIANG YAO ET AL: "Ciliotoxicity of intranasal formulations: Menthol enantiomers" CHIRALITY (1993), 5(2), 58-60 , XP002112680
- SIGMA: "Sigma Catalogue" janvier 1998 (1998-01) , SIGMA XP002112681 page 562 page 1689
- PATENT ABSTRACTS OF JAPAN vol. 016, no. 473 (C-0991), 2 octobre 1992 (1992-10-02) -& JP 04 173726 A (NIHON ZETOTSUKU KK;OTHERS: 01), 22 juin 1992 (1992-06-22) -& DATABASE WPI Section Ch, Week 9231 Derwent Publications Ltd., London, GB; Class A10, AN 92-255640 XP002112682 & JP 04 173726 A (NIPPON ZETOKKU KK), 22 juin 1992 (1992-06-22)

## Description

La présente invention concerne une composition aqueuse ionique contenant du lévomenthol.

Le vestibule nasal contient des récepteurs sensitifs qui sont stimulés par le passage de l'air. Lorsque des modifications externes interviennent, comme par exemple l'infestation par des rhinovirus, des variations de température ou de pression atmosphérique, la muqueuse du vestibule nasal réagit et se congestionne, ce qui crée une impression de nez bouché. Cette affectation bénigne, qui est communément appelée le rhume, guérit normalement spontanément mais entraîne parfois, pendant plusieurs jours, une sensation de gêne respiratoire et un dérèglement sécrétoire de la muqueuse nasale qui peuvent engendrer un inconfort important dans de nombreuses circonstances comme par exemple sur le lieu de travail ou pour dormir convenablement.

Plusieurs traitements sont actuellement proposés. Par voie générale ou locale, ce sont les médicaments à action vasoconstrictrice. Mais leur usage, à long terme, peut provoquer des effets secondaires locaux ou généraux, liés à leur action pharmacologique, qui peuvent être graves, comme une atrophie de la muqueuse nasale ou des troubles cardiovasculaires ou de la prostate. Un autre traitement consiste dans l'administration locale d'huiles essentielles et/ou de dérivés aromatiques terpéniques. Mais leur action décongestionnante ou antiseptique locale, souvent revendiquée, n'est pas scientifiquement prouvée, et leur usage, seuls ou associés, peut se révéler dangereux pour la santé en risquant de provoquer des irritations locales avec les huiles essentielles ou des troubles du système nerveux central, comme par exemple des convulsions chez l'enfant avec le camphre. Le risque est augmenté lorsque ces préparations contiennent de l'alcool, même en faible proportion, ou des substances antiseptiques, utilisées pour éviter les contaminations microbiennes mais qui peuvent être irritantes pour la muqueuse nasale. Les nourrissons, les jeunes enfants ou les sujets âgés sont tout particulièrement exposés à tous ces risques.

Le menthol naturel est un alcool secondaire de l'essence de menthe qui contient du lévomenthol mais également de la menthone. Le menthol synthétique ou *dl* menthol est un alcool obtenu par divers procédés de synthèse mais on peut également isoler ou synthétiser le l-menthol ou lévomenthol qui est l'isomère le plus actif pour l'aromatisation (cf. Codex 1965 - DORVAULT L'OFFICINE, Vigot, 23^{eme} ed. p.1055 - MARTINDALE, THE EXTRA PHAMARCOPOEIA, The Pharmaceutical Press, 30^{th} ed. p. 1386).

On a déjà proposé d'utiliser du menthol, voire même du lévomenthol dans des compositions destinées à décongestionner la muqueuse nasale.

Toutefois, le menthol étant connu pour sa faible solubilité dans les solutions aqueuses, on utilise différents artifices pour l'utiliser à des concentrations supérieures à sa limite de solubilité dans l'eau, soit en l'incorporant dans des émulsions soit en introduisant différents produits organiques tels qu'une huile ou un alcool pour améliorer sa dispersion.

La présente invention résulte d'une démarche inverse puisque l'inventeur a maintenant découvert qu'il était possible de préparer des compositions particulièrement efficaces en utilisant du lévomenthol à une concentration qui ne dépasse pas la limite de solubilité de ce dernier, à condition de le dissoudre dans un milieu aqueux suffisamment ionique, et plus précisément un milieu au moins isotonique, et de préférence même assez nettement hypertonique.

On rappellera qu'en physiologie, on qualifie d'isotonique une solution exerçant une pression osmotique égale à celle du plasma sanguin normal, par exemple le soluté physiologique de NaCl à 9 g par litre. Une solution hypertonique se dit d'une solution qui exerce une pression osmotique supérieure à celle du plasma sanguin normal (exemple : soluté de NaCl dont la concentration est supérieure à 9g par litre.). C'est dans ce sens qu'il faut comprendre les termes iso- ou hypertonique.

Dans la mesure où, comme cela ressort de l'exposé qui suit, on vise ici des solutions contenant éventuellement d'autres ions que ceux constitutifs du chlorure de sodium, on se référera pour définir le comportement osmotique des solutions ioniques de l'invention à leur osmolarité.

On désigne par osmolarité d'une solution la concentration des particules osmotiquement actives, comme les ions, dans cette solution. En physiologie, les solutions ayant une osmolarité égale à celle du plasma sanguin sont couramment dites isoosmotiques. Celles ayant une osmolarité supérieures sont dites hyperosmotiques

Plus précisément, l'invention concerne une nouvelle composition sous forme de solution aqueuse, notamment destinée à soulager la sensation de gêne respiratoire et/ou à favoriser la régénération naturelle de la muqueuse du vestibule nasal. Cette solution est essentiellement constituée d'une solution de lévomenthol dans une solution ionique aqueuse présentant une osmolarité au moins égale à celle d'une solution aqueuse de chlorure de sodium à 9 g/l et dans laquelle le lévomenthol est dissous, ladite solution ionique aqueuse étant choisie dans le groupe constitué de l'eau de mer pure ou diluée et des solutions aqueuses de chlorure de sodium contenant en outre éventuellement au moins un sel contenu naturellement dans l'eau de mer, le lévomenthol se trouvant à une concentration comprise entre 10 et 250 mg/l.

Cette composition présente le double avantage de réaliser :
1) En premier lieu. une action de lavage de la muqueuse nasale congestionnée par une solution aqueuse ionique hypertonique ou hyperosmotique, ou au minimum isotonique ou isoosmotique Cette action de lavage élimine les mucosités présentes sur la muqueuse congestionnée et provoque un effet osmotique, ce qui facilite la régénération naturelle de la muqueuse du vestibule nasal. Cette solution ionique aqueuse peut être, soit de l'eau de mer pure ou diluée, soit de l'eau contenant du chlorure de sodium et, le cas échéant, d'autres sels contenus naturellement dans l'eau de mer.
   La concentration en chlorure de sodium, de la solution choisie, est de préférence égale ou supérieure à 9 grammes par litre, concentration dite isotonique au sang ou aux larmes.
2) En deuxième lieu, et de façon complémentaire, une action sur les thermo-récepteurs ou récepteurs du froid, de la muqueuse du vestibule nasal. Cette action est obtenue par l'emploi du lévomenthol, seul isomère du menthol à être actif sur les thermo-récepteurs, ou récepteurs du froid, de la muqueuse du vestibule nasal. Le lévomenthol agit au niveau du flux des ions calcium à travers la membrane cellulaire de ces récepteurs. Un effet rafraîchissant est ainsi instantanément ressenti et procure une sensation de facilité à respirer qui permet de diminuer la sensation de gêne respiratoire due à la congestion nasale, dans l'attente d'une régénération naturelle de la muqueuse du vestibule nasal et sans utilisation d'autres substances ou de médicaments qui pourraient être dangereux.
   Ainsi donc, la présente invention réalise une véritable synergie des effets de ses deux éléments constitutifs, pour arriver à un résultat particulièrement remarquable par son effet immédiat.
   En effet, l'action du lévomenthol est optimisée :
   (i) par le lavage, par une solution ionique, de la muqueuse du vestibule nasal qui se trouve dégagée des mucosités, ce qui rend les thermo-récepteurs plus facilement accessibles au lévomenthol, et
   (ii) par l'absence, dans la composition, de substances associées, de type terpènes ou huiles essentielles ou de toutes autres substances, qui peuvent diminuer l'accès du lévomenthol à ces thermo-récepteurs.

On peut cependant envisager l'addition d'une substance non active sur la muqueuse du vestibule nasal pour, si nécessaire, stabiliser le pH de la composition.

Comme exposé précédemment, il est important que la composition présente une teneur en ions lui conférant une pression osmotique au moins égale à celle d'une composition isotonique de chlorure de sodium.

Par contre, une concentration trop élevée en ions risquerait de conduire à une composition trop irritante pour la muqueuse nasale.

C'est pourquoi les compositions ioniques de l'invention présentent avantageusement une osmolarité équivalente à celle d'une solution de chlorure de sodium comprise entre 9 et 30 g/l, de préférence comprise entre 12 et 18 g/l.

Selon une variante préférée de l'invention, la solution ionique est de l'eau de mer pure ou diluée présentant une osmolarité telle que définie ci-dessus.

Selon une autre variante, on remplacera l'eau de mer par une solution dite eau de mer synthétique contenant du chlorure de sodium et éventuellement d'autres sels naturellement contenus dans l'eau de mer naturelle.

Toutes ces solutions, qu'il s'agisse d'eau de mer naturelle ou synthétique contiendront une teneur totale en ions telle qu'elles aient l'osmolarité précédemment définie.

Parmi les ions introduits sous forme de sels dans l'eau de mer synthétique, on citera à titre d'exemple ceux du cuivre, du zinc, de l'iode, du magnésium, du manganèse, du fluor, du sélénium ainsi que les ions trouvés à l'état de trace dans l'eau de mer tels que ceux du nickel, de l'argent ou du cobalt.

Ces ions seront par exemple introduits sous l'une des formes suivantes :
- sulfate de cuivre,
- chlorure de zinc,
- iodure de potassium,
- sulfate de magnésium.
- chlorure de manganèse,
- fluorure de sodium,
- chlorure de sélénium,

Les différents ions introduits seront de préférence introduits à des concentrations égales ou inférieures à celles habituellement rencontrées dans l'eau de mer ou compatibles avec les normes en vigueur de potabilité des eaux de boissons ou d'acceptabilité pour la composition des eaux minérales.

Le lévomenthol se trouve dans les compositions de l'invention à des teneurs où il est en solution, cela, sans qu'il soit nécessaire d'ajouter des produits destinés à améliorer sa solubilité ou sa dispersabilité. Ceci constitue une différence essentielle avec les compositions de l'art antérieur.

A cet effet, la concentration du lévomenthol dans les compositions de l'invention est avantageusement comprise entre 30 et 200 mg/l, de préférence entre 100 et 180 mg/l.

Les deux constituants, solution ionique aqueuse et lévomenthol, sont dits constituants essentiels de la composition de la présente invention en ce sens que c'est à leur seule présence qu'est liée l'activité de la composition. La présence d'huile ou d'alcool, en vue notamment d'augmenter la limite de solubilité du lévomenthol dans la composition est exclue. Une telle présence pourrait d'ailleurs être néfaste.

Toutefois, on pourra admettre dans la composition la présence d'un tampon destiné à ajuster le pH à des valeurs comprises entre 6 et 12. de préférence entre 8 et 10.

A titre d'exemples de solutions tampons acceptables, on citera celles qui sont bien tolérées par la muqueuse du vestibule nasal et qui sont susceptibles de maintenir la solution en milieu légèrement alcalin, par exemple les solutions tampons à base de bicarbonate ou de phosphate de sodium.

Un avantage des compositions de l'invention est que, du fait de leur simplicité, elles peuvent être préparées dans des conditions très économiques, par simple mélange et agitation de leurs constituants. Ce mélange peut ensuite être suivi d'une étape de filtration, éventuellement d'une filtration stérilisante.

Un autre avantage des compositions de l'invention est qu'elles ne nécessitent pas l'addition d'un antiseptique ou d'un conservateur additionnel; ce qui diminue encore tout risque de caractère irritant.

Les étapes classiques de stérilisation et d'aseptisation notamment par des moyens thermiques ou par irradiation s'avèrent tout à fait suffisantes pour obtenir des compositions parfaitement fiables.

La composition de l'invention présente l'avantage de pouvoir être fabriquée industriellement de façon aseptique ou stérile, et sans subir de contamination microbienne externe qui pourrait se révéler dangereuse pour l'utilisateur.

Elle est avantageusement présentée dans un dispositif approprié pour la pulvérisation ou la nébulisation nasale.

A titre d'exemple, le dispositif vide peut être rempli avec la composition qui aura subi préalablement une filtration stérilisante. Le dispositif sera ensuite fermé ou serti, de façon à y apposer la valve ou le dispositif qui assurera la pulvérisation ou la nébulisation. L'ensemble de ces opérations peut se dérouler en milieu aseptique ou stérile.

Il peut se révéler utile de pulvériser ou de nébuliser des microdoses de ces composition, de façon à répéter plusieurs fois par jour ces opérations pour assurer le bien-être de l'utilisateur sans risque pour sa santé.

Selon une autre de ses caractéristiques essentielles, l'invention concerne l'utilisation d'une composition sous forme d'une solution contenant de 10 à 250 mg/l de lévomenthol en solution aqueuse dans de l'eau de mer pure ou diluée ou dans une solution aqueuse contenant du chlorure de sodium et éventuellement enrichi en au moins un ion contenu naturellement dans l'eau de mer, ladite solution présentant une osmolarité correspondant à celle d'une solution aqueuse de chlorure de sodium contenant de 9 à 30 g/l, de préférence 12 à 18 g de chlorure de sodium, pour la préparation d'une composition destinée à soulager la sensation de gêne respiratoire et/ou à favoriser la régénération naturelle de la muqueuse du vestibule nasal.

Selon une autre de ses caractéristiques essentielles, l'invention concerne un procédé de traitement du rhume selon lequel on administre par pulvérisation ou nébulisation nasale la composition précédemment décrite, de façon à soulager la sensation de gêne respiratoire et/ou à favoriser la régénération naturelle de la muqueuse du vestibule nasal.

Selon une autre caractéristique, la composition de l'invention peut être pulvérisée ou nébulisée, à l'aide d'un dispositif approprié, dans le conduit auditif externe, dans le but de nettoyer ce conduit et d'éliminer les impuretés qui s'y trouvent et qui, en s'accumulant, favorisent la formation de bouchons de cérumen.

Selon une autre de ses caractéristiques essentielles, l'invention concerne l'utilisation d'eau de mer éventuellement diluée pour obtenir un osmolarité équivalente à celle d'une solution de chlorure de sodium contenant de 9 à 30 g/l, et de préférence 12 à 18 g/l, ou d'une solution de chlorure de sodium contenant en outre éventuellement au moins un des sels naturellement contenus dans l'eau de mer et présentant une osmolarité équivalente à celle d'une solution aqueuse de chlorure de sodium contenant de 9 à 30 g par litre, de préférence de 12 à 18 g de chlorure de sodium, comme agent destiné à améliorer la sensation de fraîcheur apportée par le lévomenthol au niveau des récepteurs du froid du vestibule nasal.

Toutes les compositions précédemment décrites permettent d'observer cet effet de synergie entre les deux constituants essentiels de la composition et tout particulièrement dans le cas de concentrations en lévomenthol comprises entre 10 et 250 mg/l, de préférence entre 30 et 200 mg/l, de préférence encore entre 100 et 180 mg/l.

Les exemples qui suivent sont donnés à titre non limitatif. Les compositions qui y sont décrites sont obtenues par mélange de leurs constituants, filtration, stérilisation, conditionnement en flacon nébuliseur ou pulvérisateur, avec ou sans gaz propulseur, ou par tout autre procédé permettant l'administration de la composition dans le vestibule nasal.

### Exemple 1 :

| | |
|---|---|
| Lévomenthol | 9 mg |
| Eau de mer | 50 ml |

### Exemple 2 :

| | |
|---|---|
| Lévomenthol | 12 mg |
| Eau purifiée | 30 ml |
| Eau de mer | 70 ml |

### Exemple 3 :

| | |
|---|---|
| Lévomenthol | 1 mg |
| Chlorure de sodium | 135 mg |
| Eau purifiée | 15 ml |

### Exemple 4:

| | |
|---|---|
| Lévomenthol | 0,5 mg |
| Chlorure de sodium | 350 mg |
| Eau purifiée | 15 ml |

### Exemple 5 :

| | |
|---|---|
| Lévomenthol | 15 mg |
| Sulfate de magnésium | 5 mg |
| Fluorure de sodium | 4 mg |
| Chlorure de sélénium | 3 mg |
| Chlorure de sodium | 1,2 mg |
| Eau purifiée | 100 ml |

### Exemple 6 :

| | |
|---|---|
| Lévomenthol | 12 mg |
| Sulfate de cuivre | 4 mg |
| Chlorure de manganèse | 2 mg |
| Chlorure de sodium | 1,4 mg |
| Eau purifiée | 100 ml |

### Exemple 7 :

| | |
|---|---|
| Lévomenthol | 18 mg |
| Chlorure de zinc | 1,5 mg |
| Chlorure de sodium | 1,8 mg |
| Eau purifiée | 100 ml |

Les compositions décrites dans les exemples 1 à 7 ci-dessus sont administrées, en cas de rhume ou de nez congestionné, à raison de 1 à 10 nébulisations de 25 à 150 microlitres par jour dans chaque narine.

## Revendications

1. Composition sous forme de solution aqueuse, notamment destinée à soulager la sensation de gêne respiratoire et/ou à favoriser la régénération naturelle de la muqueuse du vestibule nasal, **caractérisée en ce qu'**elle est essentiellement constituée d'une solution de lévomenthol dans une solution ionique aqueuse présentant une osmolarité au moins égale à celle d'une solution aqueuse de chlorure de sodium à 9 g/l et dans laquelle le lévomenthol est dissous, ladite solution ionique aqueuse étant choisie dans le groupe constitué de l'eau de mer pure ou diluée et des solutions aqueuses de chlorure de sodium contenant en outre éventuellement au moins un sel naturellement contenu dans l'eau de mer, le lévomenthol se trouvant à une concentration comprise entre 10 et 250 mg/l.

2. Composition selon la revendication 1, **caractérisée en ce que** ladite solution ionique présente une osmolarité équivalente à celle d'une solution de chlorure de sodium contenant de 9 à 30 g/l.

3. Composition selon l'une des revendications 1 à 2, **caractérisée en ce que** ladite solution ionique présente une osmolarité équivalente à celle d'une solution de chlorure de sodium contenant de 12 à 18 g/l.

4. Composition selon l'une des revendications 1 à 3, **caractérisée en ce que** le lévomenthol est en solution dans de l'eau de mer pure ou diluée présentant une osmolarité équivalente à celle d'une solution aqueuse de chlorure de sodium contenant de 9 à 30 g de chlorure de sodium par litre, de préférence de 12 à 18 g/l.

5. Composition selon l'une des revendications 1 à 3, **caractérisée en ce que** le lévomenthol est en solution dans une solution de chlorure de sodium contenant de 9 à 30 g par litre, de préférence de 12 à 18 g par litre.

6. Composition selon l'une des revendications 1 à 3, **caractérisée en ce que** le lévomenthol est en solution dans une solution aqueuse contenant du chlorure de sodium, ladite solution contenant en outre au moins un ion choisi dans le groupe constitué des ions du cuivre, du zinc, de l'iode, du magnésium, de l'argent, du manganèse, du fluor, du nickel, du cobalt, du sélénium, ladite solution présentant une osmolarité équivalente à celle d'une solution de chlorure de sodium contenant de 9 à 30 g/l, de préférence 12 à 18 g/l.

7. Composition selon l'une des revendications 1 à 6, **caractérisée en ce qu'**elle contient de 30 à 200 mg/l de lévomenthol.

8. Composition selon l'une des revendications 1 à 7, **caractérisée en ce qu'**elle contient de 100 à 180 mg/l de lévomenthol.

9. Composition selon l'une des revendications 1 à 8, **caractérisée en ce qu'**elle contient en outre un tampon destiné à ajuster le pH.

10. Utilisation d'une composition sous forme d'une solution contenant de 10 à 250 mg/l de lévomenthol en solution aqueuse dans de l'eau de mer pure ou diluée ou dans une solution aqueuse contenant du chlorure de sodium et éventuellement enrichi en au moins un ion contenu naturellement dans l'eau de mer, ladite solution présentant une osmolarité correspondant à celle d'une solution aqueuse de chlorure de sodium contenant de 9 à 30 g/l, de préférence 12 à 18 g de chlorure de sodium, pour la préparation d'une composition destinée à soulager la sensation de gêne respiratoire et/ou à favoriser la régénération naturelle de la muqueuse du vestibule nasal.

11. Utilisation d'une composition sous forme d'une solution contenant de 10 à 250 mg/l de lévomenthol en solution aqueuse dans de l'eau de mer pure ou diluée ou dans une solution aqueuse contenant du chlorure de sodium et éventuellement enrichi en au moins un ion contenu naturellement dans l'eau de mer, ladite solution présentant une osmolarité correspondant à celle d'une solution aqueuse de chlorure de sodium contenant de 9 à 30 g/l, de préférence 12 à 18 g de chlorure de sodium, pour la préparation d'une composition destinée à nettoyer le conduit auditif externe et à éliminer les impuretés qui s'y trouvent.

12. Utilisation d'eau de mer éventuellement diluée pour obtenir un osmolarité équivalente à celle d'une solution de chlorure de sodium contenant de 9 à 30 g/l., ou d'une solution de chlorure de sodium contenant en outre éventuellement au moins un des sels naturellement contenus dans l'eau de mer et présentant une osmolarité équivalente à celle d'une solution aqueuse de chlorure de sodium contenant de 9 à 30 g/l, de préférence de 12 à 18 g/l comme agent destiné à améliorer la sensation de fraîcheur apportée par le lévomenthol au niveau des récepteurs du froid du vestibule nasal.

13. Utilisation selon la revendication 11, **caractérisée en ce que** ladite solution contient de 10 à 250 mg/l de lévomenthol, de préférence de 30 à 200 mg/l, de préférence encore de 100 à 180 mg/l.

## Patentansprüche

1. Zusammensetzung in Form einer wässrigen Lösung, die insbesondere das Gefühl der Behinderung der Atmung erleichtern und/oder die natürliche Regeneration der Nasenhöhlenschleimhaut fördern soll, **dadurch gekennzeichnet, dass** sie im wesentlichen besteht aus einer Lösung von Levomenthol (linksdrehendern Menthol) in einer wässrigen ionischen Lösung, die eine Osmolarität aufweist, die mindestens gleich derjenigen einer wässrigen Natriumchloridlösung mit 9 g/l Natriumchlorid ist und in der das Levomenthol gelöst ist, wobei die genannte wässrige ionische Lösung ausgewählt ist aus der Gruppe, die besteht aus reinem oder verdünntem Meerwasser und wässrigen Natriumchloridlösungen, die außerdem gegebenenfalls mindestens ein Salz enthalten, das in natürlicher Weise in Meerwasser enthalten ist, wobei das Levomenthol in einer Konzentration zwischen 10 und 250 mg/l vorliegt.

2. Zusammensetzung nach Anspruch 1, **dadurch gekennzeichnet, dass** die genannte ionische Lösung eine Osmolarität aufweist, die derjenigen einer Natriumchloridlösung entspricht, die 9 bis 30 g/l Natriumchlorid enthält.

3. Zusammensetzung nach einem der Ansprüche 1 bis 2, **dadurch gekennzeichnet, dass** die genannte ionische Lösung eine Osmolarität aufweist, die derjenigen einer Natriumchloridlösung entspricht, die 12 bis 18 g/l Natriumchlorid enthält.

4. Zusammensetzung nach einem der Ansprüche 1 bis 3, **dadurch gekennzeichnet, dass** das Levomenthol in reinem oder verdünntem Meerwasser gelöst ist, das eine Osmolarität aufweist, die derjenigen einer wässrigen Natriumchloridlösung entspricht, die 9 bis 30 g, vorzugsweise 12 bis 18 g Natriumchlorid pro Liter enthält.

5. Zusammensetzung nach einem der Ansprüche 1 bis 3, daduch gekennzeichnet, dass das Levomenthol in einer Natriumchloridlösung gelöst ist, die 9 bis 30 g, vorzugsweise 12 bis 18 g Natriumchlorid pro Liter enthält.

6. Zusammensetzung nach einem der Ansprüche 1 bis 3, **dadurch gekennzeichnet, dass** das Levomenthol in einer Natriumchlorid enthaltenden wässrigen Lösung gelöst ist, die außerdem mindestens ein Ion, ausgewählt aus der Gruppe, die besteht aus Kupfer-, Zink-, Iod-, Magnesium-, Silber-, Mangan-, Fluor-, Nickel-, Kobalt- und Selen-Ionen, enthält, wobei die genannte Lösung eine Osmolarität aufweist, die derjenigen einer Natriumchloridlösung entspricht, die 9 bis 30 g, vorzugsweise 12 bis 18 g Natriumchlorid pro Liter enthält.

7. Zusammensetzung nach einem der Ansprüche 1 bis 6, **dadurch gekennzeichnet, dass** sie 30 bis 200 mg/l Levomenthol enthält.

8. Zusammensetzung nach einem der Ansprüche 1 bis 7, **dadurch gekennzeichnet, dass** sie 100 bis 180 mg/l Levomenthol enthält.

9. Zusammensetzung nach einem der Ansprüche 1 bis 8, **dadurch gekennzeichnet, dass** sie außerdem einen Puffer zur Einstellung des pH-Wertes enthält.

10. Verwendung einer Zusammensetzung in Form einer Lösung, die 10 bis 250 mg/l Levomenthol in wässriger Lösung in reinem oder verdünntem Meerwasser oder in einer Natriumchlorid enthaltenden wässrigen Lösung enthält, die gegebenenfalls angereichert ist an mindestens einem Ion, das in natürlicher Weise in Meerwasser enthalten ist, wobei die genannte Lösung eine Osmolarität aufweist, die derjenigen einer wässrigen Natriumchloridlösung entspricht, die 9 bis 30 g/l, vorzugsweise 12 bis 18 g/l Natriumchlorid enthält, zur Herstellung einer Zusammensetzung zur Beseitigung des Gefühls einer behinderten Atmung und/oder zur Förderung der natürlichen Regeneration der Nasenhöhlenschleimhaut.

11. Verwendung einer Zusammensetzung in Form einer Lösung, die 10 bis 250 mg/l Levomenthol in wässriger Lösung enthält, in reinem oder verdünntem Meerwasser oder in einer Natriumchlorid enthaltenden wässrigen Lösung, die gegebenenfalls an mindestens einem Ion angereichert ist, das in natürlicher Weise in Meerwasser enthalten ist, wobei die genannte Lösung eine Osmolarität aufweist, die derjenigen einer wässrigen Natriumchloridlösung entspricht, die 9 bis 30 g/l, vorzugsweise 12 bis 18 g/l Natriumchlorid enthält, zur Herstellung einer Zusammensetzung für die Reinigung des äußeren Gehörganges und die Entfermung von sich darin befindenden Verunreinigungen.

12. Verwendung von gegebenenfalls verdünntem Meerwasser zur Einstellung einer Osmolarität, die derjenigen einer Natriumchloridlösung, die 9 bis 30 g/l Natriumchlorid enthält, oder einer Natriumchloridlösung entspricht, die außerdem mindestens eines der Salze enthält, die in natürlicher Weise in Meerwasser enthalten sind, und eine Osmolarität aufweist, die derjenigen einer wässrigen Natriumchloridlösung entspricht, die 9 bis 30 g/l, vorzugsweise 12 bis 18 g/l Natriumchlorid enthält, als Mittel zur Verbesserung des Frischegefühls, das durch Lovomenthol vermittelt wird, im Bereich der Kälterezeptoren der Nasenhöhle.

13. Verwendung nach Anspruch 11, **dadurch gekennzeichnet, dass** die genannte Lösung 10 bis 250 mg/l, vorzugsweise 30 bis 200 mg/l, besonders bevorzugt 100 bis 180 mg/l Levomenthol enthält.

## Claims

1. A composition in the form of an aqueous solution, notably intended for relieving the sensation of respiratory discomfort and/or for promoting the natural regeneration of the mucous membranes of the nasal cavity, **characterised in that** it is essentially constituted of a levomenthol solution in an aqueous ionic solution having an osmolarity at least equal to that of an aqueous solution of sodium chloride at 9 g/l and in which the levomenthol is dissolved, said aqueous ionic solution being selected from the group constituted of pure or diluted sea water and aqueous sodium chloride solutions optionally further containing at least one salt naturally contained in sea water, the levomenthol being found at a concentration between 10 and 250 mg/l.

2. The composition according to claim 1, **characterised in that** said ionic solution has an osmolarity which is equivalent to that of a solution of sodium chloride containing from 9 to 30 g/l.

3. The composition according to one of claims 1 to 2, **characterised in that** said ionic solution has an osmolarity which is equivalent to that of a solution of sodium chloride containing from 12 to 18 g/l.

4. The composition according to one of claims 1 to 3, **characterised in that** the levomenthol is in solution in pure or diluted sea water having an osmolarity which is equivalent to that of an aqueous solution of sodium chloride containing from 9 to 30 g of sodium chloride per litre, preferably from 12 to 18 g/l.

5. The composition according to one of claims 1 to 3, **characterised in that** the levomenthol is in solution in a solution of sodium chloride containing from 9 to 30 g per litre, preferably from 12 to 18 g per litre.

6. The composition according to one of claims 1 to 3, **characterised in that** the levomenthol is in solution in an aqueous solution containing sodium chloride, said solution further containing at least one ion selected from the group constituted of copper, zinc, iodine, magnesium, silver, manganese, fluorine, nickel, cobalt, selenium ions, said solution having an osmolarity which is equivalent to that of a solution of sodium chloride containing from 9 to 30 g/l, preferably 12 to 18 g/l.

7. The composition according to one of claims 1 to 6, **characterised in that** it contains from 30 to 200 mg/l of levomenthol.

8. The composition according to one of claims 1 to 7, **characterised in that** it contains from 100 to 180 mg/l of levomenthol.

9. The composition according to one of claims 1 to 8, **characterised in that** it further contains a buffer intended for adjusting the pH.

10. Use of a composition in the form of a solution containing from 10 to 250 mg/l of levomenthol in aqueous solution in pure or diluted sea water or in an aqueous solution containing sodium chloride and optionally enriched with at least one ion naturally contained in sea water, said solution having an osmolarity corresponding to that of an aqueous solution of sodium chloride containing from 9 to 30 g/l, preferably 12 to 18 g of sodium chloride, for preparing a composition intended for relieving the sensation of respiratory discomfort and/or for promoting the natural regeneration of the mucous membranes of the nasal cavity.

11. Use of a composition in the form of a solution containing from 10 to 250 mg/l of levomenthol in aqueous solution in pure or diluted sea water or in an aqueous solution containing sodium chloride and optionally enriched with at least one ion naturally contained in sea water, said solution having an osmolarity corresponding to that of an aqueous solution of sodium chloride containing from 9 to 30 g/l, preferably 12 to 18 g of sodium chloride, for preparing a composition intended for cleaning the external auditory canal and for removing impurities found therein.

12. Use of optionally diluted sea water for obtaining an osmolarity which is equivalent to that of a solution of sodium chloride containing from 9 to 30 g/l., or of a solution of sodium chloride optionally further containing at least one of the salts contained naturally in sea water and having an osmolarity which is equivalent to that of an aqueous solution of sodium chloride containing from 9 to 30 g/l, preferably from 12 to 18 g/l as an agent intended for improving the sensation of freshness brought about by the levomenthol at the cold receptors of the nasal cavity.

13. Use according to claim 11, **characterised in that** said solution contains from 10 to 250 mg/l of levomenthol, preferably from 30 to 200 mg/l, more preferably from 100 to 180 mg/l.
